(19) Europäisches Patentamt
European Patent Office
Office européen des brevets

(11) Veröffentlichungsnummer: **0 391 849 B1**

(12) **EUROPÄISCHE PATENTSCHRIFT**

(45) Veröffentlichungstag der Patentschrift: **19.04.95**

(51) Int. Cl.[6]: **C07D 401/12**, A01N 43/707

(21) Anmeldenummer: **90810252.8**

(22) Anmeldetag: **29.03.90**

# (54) Pyridyl-methylenamino-1,2,4-triazinone.

(30) Priorität: **06.04.89 CH 1275/89**

(43) Veröffentlichungstag der Anmeldung:
**10.10.90 Patentblatt 90/41**

(45) Bekanntmachung des Hinweises auf die Patenterteilung:
**19.04.95 Patentblatt 95/16**

(84) Benannte Vertragsstaaten:
**AT BE CH DE DK ES FR GB IT LI NL**

(56) Entgegenhaltungen:
**EP-A- 0 314 615**
**DE-A- 1 795 785**

(73) Patentinhaber: **CIBA-GEIGY AG**
**Klybeckstrasse 141**
**CH-4002 Basel (CH)**

(72) Erfinder: **Kristiansen, Odd, Dr.**
**Delligrabenstrasse 7**
**CH-4313 Möhlin (CH)**
Erfinder: **Böger, Manfred**
**Wilhelm Glockstrasse 14**
**D-7858 Weil am Rhein 5 (DE)**
Erfinder: **Kristinsson, Haukur, Dr.**
**Kreuzackerweg 19**
**CH-4103 Bottmingen (CH)**
Erfinder: **Maienfisch, Peter, Dr.**
**Traugott Meyer-Strasse 5**
**CH-4147 Aesch (CH)**

**EP 0 391 849 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue insektizid wirksame N-Amino-1,2,4-triazinone, Verfahren zu ihrer Herstellung, Mittel, welche diese Aminotriazinone enthalten, die Verwendung dieser Verbindungen in der Schädlingsbekämpfung sowie ein Verfahren zur Schädlingsbekämpfung mit diesen Verbindungen.

Die erfindungsgemässen Aminotriazinone entsprechen der Formel

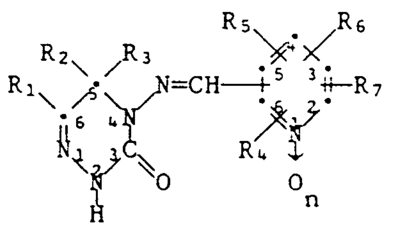

(I),

worin
$R_1$ $C_1$-$C_{12}$-Alkyl oder $C_3$-$C_7$-Cycloalkyl,
$R_2$ und $R_3$ voneinander unabhängig Wasserstoff oder $C_1$-$C_6$-Alkyl,
$R_4$, $R_5$, $R_6$ und $R_7$ voneinander unabhängig Wasserstoff, Halogen, $C_1$-$C_3$-Alkyl,

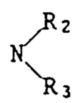
,

$C_1$-$C_3$-Alkoxy oder $C_1$-$C_3$-Alkylthio und n 0 oder 1
bedeuten, und können auch als Salze vorliegen, mit der Massgabe, dass n für 1 steht, wenn gleichzeitig der Pyridinring über die 3-Position an die Methylidengruppe gebunden ist und $R_4$, $R_5$, $R_6$ und $R_7$ für Wasserstoff stehen.

Salze von Verbindungen der Formel I sind Säueradditionssalzen. Zur Bildung solcher Salze eignen sich sowohl organische als auch anorganische Säuren. Beispiele solcher Säuren sind u.a. Chlorwasserstoffsäure, Bromwasserstoffsäure, Salpetersäure, verschiedene Phosphorsäuren, Schwefelsäure, Essigsäure, Propionsäure, Buttersäure, Valeriansäure, Oxalsäure, Malonsäure, Maleinsäure, Fumarsäsure, Milchsäure, Weinsäure oder Salicylsäure.

Die als Substituenten oder als Bestandteile von Substituenten in Betracht kommenden Alkylgruppen können geradkettig oder verzweigt sein. Als Beispiele solcher Alkyle seien Methyl, Aethyl, Propyl, Isopropyl, Butyl, i-Butyl, sek.Butyl, tert.Butyl oder Pentyl, Hexyl, Octyl, Decyl, Dodecyl usw. und ihre Isomeren genannt.

Die als Substituenten in Betracht kommenden Alkoxy- und Alkylthioreste können geradkettig oder verzweigt sein. Geeignete Beispiele solcher Substituenten sind u.a. Methoxy, Methylthio, Ethoxy, Ethylthio und Propoxy.

Bei den als Substituenten in Betracht kommenden Cycloalkylgruppen handelt es sich beispielsweise um Cyclopropyl, Cyclobutyl, Cyclopentyl oder Cyclohexyl.

Bei den als Substituenten in Betracht kommenden Halogenen handelt es sich sowohl um Fluor und Chlor, als auch um Brom und Jod, wobei Fluor und Chlor bevorzugt werden.

Unter den Verbindungen der Formel I stehen solche im Vordergrund, worin $R_1$ $C_1$-$C_4$-Alkyl bedeutet; solche, worin $R_1$ Methyl, Ethyl, Isopropyl oder Cyclopropyl bedeutet; und solche, worin n für 1 steht.

Bevorzugt sind auch die Verbindungen der Formel Ia

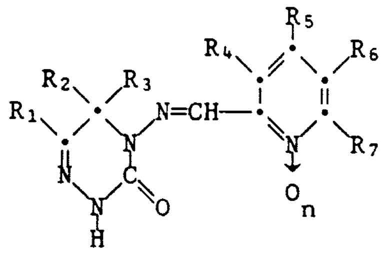

(Ia),

worin $R_1$ bis $R_7$ und n die vorstehend angegebenen Bedeutungen aufweisen.

Vorteilhafterweise bedeuten in den obigen Formeln I und Ia $R_2$ und $R_3$ unabhhängig voneinander Wasserstoff oder Methyl, vorzugsweise Wasserstoff.

Unter den Verbindungen der Formel I sind auch jene der Formel Ib

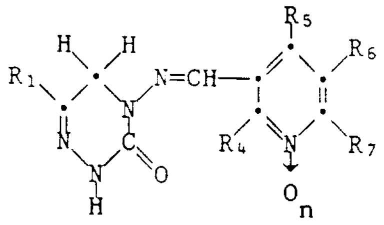

(Ib)

hervorzuheben, in welcher $R_1$, $R_4$ bis $R_7$ und n die vorstehend angegebenen Bedeutungen haben.

Von den Verbindungen der Formel Ib sind wegen ihrer biologischen Aktivität jene bevorzugt, bei welchen $R_1$ Methyl oder Ethyl, $R_5$ Wasserstoff, $R_4$, $R_6$ und $R_7$ unabhängig voneinander Chlor, Methyl, Methoxy oder Amino bedeuten.

Insbesondere bevorzugt sind erfindungsgemässe Verbindungen, der Formeln I, Ia und Ib worin $R_6$ Wasserstoff bedeutet; oder worin $R_4$, $R_5$, $R_6$ und $R_7$ Wasserstoff und n 1 bedeuten; oder worin $R_1$ Methyl bedeutet.

Ferner sind unter den Verbindungen der Formel Ib auch jene bevorzugt, bei welchen $R_4$, $R_5$ und $R_6$ voneinander unabhängig Methyl oder Fluor und $R_7$ Wasserstoff, Methylthio oder Dimethylamino und n 0 bedeuten.

Unter den Verbindungen der Formel I sind auch jene der Formel Ic speziell zu erwähnen

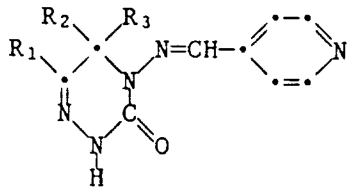

(Ic),

worin $R_1$, $R_2$, und $R_3$ die vorstehend angegebenen Bedeutungen besitzen.

Das 2,3,4,5-Tetrahydro-3-oxo-4-[(pyridin-N-oxyd-3-yl)-methylenimino]-6-methyl-1,2,4-triazin der Formel

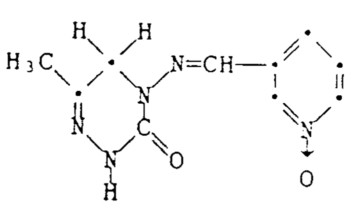

ist wegen seiner biologischen Wirkung speziell hervorzuheben.

Die erfindungsgemässen Verbindungen der Formel I können nach im Prinzip bekannten Verfahren hergestellt werden, indem man z.B. ein Aminotriazinon der Formel

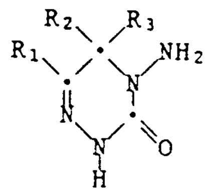

(II),

worin
$R_1$ $C_1$-$C_{12}$-Alkyl oder $C_3$-$C_7$-Cycloalkyl und
$R_2$ und $R_3$ voneinander unabhängig Wasserstoff oder $C_1$-$C_6$-Alkyl bedeuten, mit einem Aldehyd der Formel

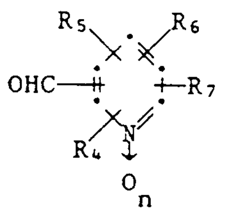

(III),

worin
$R_4$, $R_5$, $R_6$ und $R_7$ voneinander unabhängig Wasserstoff, Halogen, $C_1$-$C_3$-Alkyl,

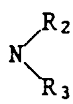

,

$C_1$-$C_3$-Alkoxy oder $C_1$-$C_3$-Alkylthio und n 0 oder 1
bedeuten, mit der Massgabe, dass n für 1 steht, wenn gleichzeitig der Pyridinring über die 3-Position an die Methylidengruppe gebunden ist und $R_4$, $R_5$, $R_6$ und $R_7$ für Wasserstoff stehen, umsetzt und das Produkt isoliert. Die erhaltenen Verbindungen können, falls erwünscht, in üblicher Weise in ihre Salze umgewandelt werden.

Das Verfahren wird im allgemeinen unter normalem Druck in Gegenwart einer katalytischen Menge einer starken Säure und in einem Lösungsmittel durchgeführt. Die Reaktionstemperatur beträgt dabei zwischen +10 und 100°C, vorzugsweise +40 bis 80°C. Als Säuren eignen sich starke anorganische Säuren wie z.B. Mineralsäuren, insbesondere Salzsäure. Als Lösungsmittel eigenen sich Alkohole, Aether und ätherartige Verbindungen, Nitrile oder auch Wasser.

Die als Ausgangsstoffe eingesetzten Aminotriazinone der Formel II sind bekannt oder können in an sich bekannter Weise, z.B. durch eine Ringumlagerung mit Hydrazinhydrat, hergestellt werden, indem man ein Oxadiazolon der Formel

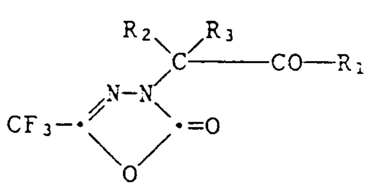

(IV)

mit Hydrazinhydrat ($H_2N$-$NH_2 \cdot H_2O$) umsetzt, wobei $R_1$, $R_2$ und $R_3$ die oben angegebenen Bedeutungen haben. Das Verfahren zur Herstellung der Aminotriazinone der Formel II wird üblicherweise unter normalem Druck und gegebenenfalls in einem Lösungsmittel durchgeführt. Die Temperatur beträgt dabei zwischen +15 und 120°C, vorzugsweise zwischen +20 und 80°C. Geeignete Lösungsmittel sind u.a. Wasser, Nitrile wie Acetonitril, Alkohole, Dioxan oder Tetrahydrofuran.

Die vorerwähnten Oxadiazolone der Formel IV können nach im Prinzip bekannten Verfahren hergestellt werden, indem man z.B. das 5-Trifluormethyl-1,3,4-oxadiazol-2(3H)-on der Formel

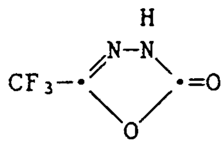

(V)

mit einem Keton der Formel

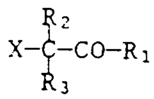

(VI)

umsetzt, wobei $R_1$, $R_2$ und $R_3$ die oben angegebenen Bedeutungen haben und X für Halogen steht. Das Verfahren zur Herstellung der Oxadiazolone der Formel IV wird bei normalem Druck in Gegenwart einer Base und in einen Lösungsmittel durchgeführt. Die Temperatur beträgt dabei zwischen 0 und +150 °C, vorzugsweise zwischen +20 und 100 °C. Als Basen eignen sich organische und anorganische Basen, wie Trimethylamin, Alkoholate, Natriumhydroxid oder Natriumhydrid. Als Lösungsmittel eigenen sich u.a. Alkohole, halogenierte Kohlenwasserstoffe, wie Chloroform, Nitrile, wie Acetonitril, Tetrahydrofuran, Dioxan, Dimethylsulfoxid oder auch Wasser.

Von den Aminotriazinonen der Formel II ist z.B. das 4-Amino-6-phenyl-1,2,4-triazin-3-on bekannt (Liebigs Annalen der Chemie, 749, 125 (1971), d.h. die Verbindung der Formel II, in der $R_1$ Phenyl, und $R_2$ und $R_3$ je Wasserstoff bedeuten. Die Verbindungen der Formeln V und VI sind bekannt oder können nach im Prinzip bekannten Verfahren hergestellt werden. Die Aldehyde der Formel III, worin n = 0 ist, sind bekannt oder können nach bekannten Verfahren hergestellt werden [Z. Chemie 184 (1970), J. Org. Chem. 46, 4836 (1981), Eur. J. Med. Chem. 12, 531 (1977), Heterocycles 26, 4836 (1981), J. Org. Chem. 53, 5320 (1988)]. Die Herstellung der Verbindungen der Formel III (n = 1) erfolgt allgemein ausgehend aus dem entsprechenden Aldehyd (n = 0) durch Umsetzung mit einem geeigneten Oxidationsmittel, wie beispielsweise m-Chlorperbenzoesäure, wobei die Aldehydgruppe mit Hilfe einer geeigneten Schutzgruppe vorübergehend geschützt wird. Für diesen Zweck kann die Aldehydgruppe beispielsweise acetalisiert werden.

Das erfindungsgemässe Verfahren eignet sich zur Darstellung der Verbindungen der, Formel I sowohl mit Pyridin-Derivaten (n = 0) als auch mit Pyridin-N-Oxiden (n = 1) als Ausgangsprodukten. Je nach Zielverbindung wird ein entsprechender freier Pyridinaldehyd (n = 0) oder dessen N-Oxid (n = 1) eingesetzt.

Es wurde nun gefunden, dass die vorliegenden Verbindungen der Formel I bei guter Pflanzenverträglichkeit eine bessere Warmblüterverträglichkeit und eine höhere Stabilität aufweisen als bekannte Phosphorsäureester und Carbamate. Sie eignen sich deshalb ausgezeichnet als Schädlingsbekämpfungsmittel, vor allem zur Bekämpfung von Pflanzen befallenden Schädlingen, insbesondere von Insekten.

Die Verbindungen der Formel I eignen sich insbesondere zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Mit Hilfe der erfindungsgemäss verwendeten Verbindungen der Formel I können vor allem pflanzenschädigende Insekten, speziell pflanzenschäigende Insekten in Zier- und Nutzpflanzungen, insbesondere in Baumwollkulturen, Gemüsekulturen, Reiskulturen und Obstkulturen bekämpft werden. In diesem Zusammenhang ist hervorzuheben, dass die genannten Verbindungen sich sowohl durch eine stark ausgeprägte systemische als auch Kontakt-Wirkung gegen saugende Insekten, vor allem gegen Insekten der Familie Aphididae (wie z.B. Aphis fabae, Aphis craccivora, Myzus persicae und Bemisia tabaci), welche sich mit herkömmlichen Mitteln nur schwierig bekämpfen lassen, auszeichnen.

Die gute pestizide Wirkung der erfindungsgemäss vorgeschlagenen Verbindungen der Formel I entspricht einer Abtötungsrate (Mortalität) von mindestens 50-60 % der erwähnten Schädlinge.

Die Wirkung der erfindungsgemäss verwendeten Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden als Schädlingsbekämpfungsmittel in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmononethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäureoder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides (oberflächenaktiven Mittels), wobei sich die %-Angaben auf das Gewicht beziehen. Während als Handelsware eher konzentrierte Mittel bevorzugt werden, verwendet der Endverbraucher in der Regel verdünnte Mittel bzw. Zubereitungen, die wesentlich geringere Wirkstoffkonzentrationen, wie z.B. 0,1 bis 1000 ppm, aufweisen. Gewöhnlich beträgt die eingesetzte Aufwandmenge der erfindungsgemässen Wirkstoffe der Formel I - insbesondere für landwirtschaftliche Kulturflächen - 0,025 bis 1,0 kg/ha, vorzugsweise 0,1 bis 0, 5 kg/ha, beispielsweise 0,1 bis 0,25 kg/ha.

Die erfindungsgemässen Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiele:

1. Herstellung der Verbindungen der Formel I und ihrer Zwischenprodukte.

Beispiel H.1: 2-Oxo-5-trifluormethyl-2,3-dihydro-1,3,4-oxadiazol-3-aceton.

15 g (0,5 Mol) einer 80%-igen NaH-Dispersion in Oel werden mit Petroläther ölfrei gewaschen und in 125 ml Dimethylformamid (DMF) vorgelegt. Zu dieser Suspension werden 77 g (0,5 Mol) 5-Trifluormethyl-1,3,4-oxadiazol-2(3H)-on in 250 ml DMF bei Raumtemperatur innerhalb 1 Stunde zugetropft und anschliessend während 3 Stunden gerührt. Dann werden 55,5 g (0,6 Mol) Chloraceton zugegeben und das Reaktionsgemisch wird während 16 Stunden bei Raumtemperatur gerührt. Nach dem Eindampfen wird der Rückstand mit 1000 ml Wasser versetzt und der feste Niederschlag abgesaugt und getrocknet. Die Titelverbindung der Formel

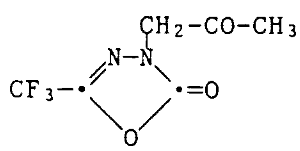

(Verbindung Nr. 1.01)
liegt in Form eines farblosen Festkörpers vor; Smp. 85 °C (Ausbeute: 96 g; 91,7 %).

Auf analoge Weise werden die folgenden Verbindungen der Formel IV hergestellt

Tabelle 1:

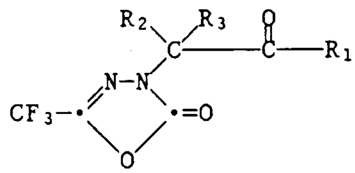

(IV)

| Verbindung Nr. | $R_1$ | $R_2$ | $R_3$ | Physik. Daten |
|---|---|---|---|---|
| 1.01 | $CH_3$ | H | H | Smp. 85°C |
| 1.02 | i-$C_3H_7$ | H | H | Smp. 74-75°C |
| 1.03 | $C(CH_3)_3$ | H | H | Smp. 67°C |
| 1.04 | $C_6H_5$ | H | H | Smp. 100-102°C |
| 1.05 | $CH_3$ | $CH_3$ | H | Oel |
| 1.06 | $CH_3$ | $CH_3$ | $CH_3$ | Oel |
| 1.07 | $C_2H_5$ | H | H | Smp. 76-77°C |
| 1.08 | 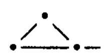 | H | H | Smp. 77-78°C |
| 1.09 | H | H | H | |
| 1.10 | $C_2H_5$ | $CH_3$ | H | |
| 1.11 | n-$C_3H_7$ | H | H | |
| 1.12 | 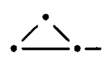 | $CH_3$ | H | |
| 1.13 | H | $CH_3$ | H | |
| 1.14 | i-$C_3H_7$ | $CH_3$ | H | |
| 1.15 | i-$C_3H_7$ | $CH_3$ | $CH_3$ | |
| 1.16 | $C(CH_3)_3$ | $CH_3$ | H | |
| 1.17 | $CH_3$ | $C_2H_5$ | H | |
| 1.18 | $CH_3$ | $C_2H_5$ | $CH_3$ | |

Tabelle 1 (Fortsetzung):

| Verbindung Nr. | $R_1$ | $R_2$ | $R_3$ | Physik. Daten |
|---|---|---|---|---|
| 1.19 | 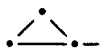 | $CH_3$ | $CH_3$ | |
| 1.20 | 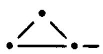 | $C_2H_5$ | $CH_3$ | |

Beispiel H.2: 2,3,4,5-Tetrahydro-3-oxo-4-amino-6-methyl-1,2,4-triazin

In 250 ml Hydrazinhydrat werden 210 g (1,0 Mol) 2-Oxo-5-trifluormethyl-2,3-dihydro-1,3,4-oxadiazol-3-aceton unter Kühlung eingetragen. Die resultierende klare braune Lösung wird nach 2-stündigem Rühren im Vakuum eingedampft und der Rückstand am Kieselgel (Methylenchlorid/Methanol 9:1) chromatographiert. Das Lösungsmittel wird abgedampft und aus dem zurückbleibenden Oel kristallisiert die Titelverbindung der Formel

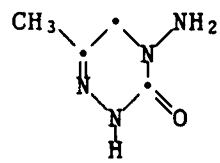

(Verbindung Nr. 2.01)
nach Zusatz von Aether; Smp. 117-119 °C (Ausbeute: 64 g; 50 %).

Auf analoge Weise werden die folgenden Verbindungen der Formel II hergestellt:

Tabelle 2:

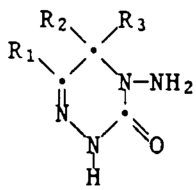 (II)

| Verbindung Nr. | $R_1$ | $R_2$ | $R_3$ | Smp. °C |
|---|---|---|---|---|
| 2.01 | $CH_3$ | H | H | 117-119 |
| 2.02 | $CH_3$ | $CH_3$ | H | 172-174 |
| 2.03 | $CH_3$ | $CH_3$ | $CH_3$ | 138-139 |
| 2.04 | $C_2H_5$ | H | H | 143-145 |
| 2.05 | $i-C_3H_7$ | H | H | 79-81 |
| 2.06 | $C(CH_3)_3$ | H | H | 148-150 |
| 2.07 | 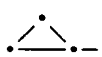 | H | H | 94-95 |
| 2.08 | $C_6H_5$ | H | H | 199-202 |
| 2.09 | $4-Cl-C_6H_4$ | H | H | 208-210 |
| 2.10 | H | H | H | |
| 2.11 | $CH_3$ | $C_2H_5$ | H | |
| 2.12 | $C_2H_5$ | $CH_3$ | $CH_3$ | |
| 2.13 | $C_2H_5$ | $CH_3$ | H | |
| 2.14 | $n-C_3H_7$ | H | H | |
| 2.15 | $n-C_3H_7$ | $CH_3$ | H | |
| 2.16 | $n-C_3H_7$ | $CH_3$ | $CH_3$ | |
| 2.17 | $i-C_3H_7$ | $CH_3$ | H | |
| 2.18 | $C(CH_3)_3$ | $CH_3$ | H | |
| 2.19 | $C(CH_3)_3$ | $CH_3$ | $CH_3$ | |

Tabelle 2 (Fortsetzung):

| Verbindung Nr. | $R_1$ | $R_2$ | $R_3$ | Smp. °C |
|---|---|---|---|---|
| 2.20 | 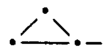 | $CH_3$ | $CH_3$ | |
| 2.21 | 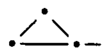 | $CH_3$ | H | |

Beispiel H.3: 2,3,4,5-Tetrahydro-3-oxo-4-[(2-methyl-pyridin-3-yl)-methylenamino]-6-methyl-1,2,4-triazin

0,5 g (4 Mmol) 2,3,4,5-Tetrahydro-3-oxo-4-amino-6-methyl-1,2,4-triazin (gelöst in 250 ml Aethanol) werden bei Raumtemperatur mit 0,48 g (4 Mmol) 2-Methylpyridin-3-carbaldehyd und 1 Tropfen konzentrierter HCl versetzt. Nach halbstündigem Rühren wird das Reaktionsgut filtriert, der abfiltrierte Festanteil mit Aether gewaschen und getrocknet. Die so erhaltene Titelverbindung der Formel

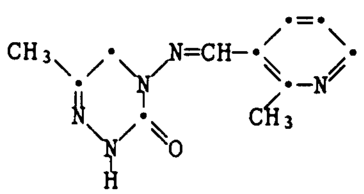

(Verbindung Nr. 4.19)
liegt als farbloser Festkörper vor; Smp. 226-229 °C (Ausbeute: 0,6 g; 64 %).

Beispiel H.4: 2,3,4,5-Tetrahydro-3-oxo-4-[(pyridin-N-oxyd-3-yl)-methylenamino]-6-methyl-1,2,4-triazin

2,56 g (0,02 Mol) 2,3,4,5-Tetrahydro-3-oxo-4-amino-6-methyl-1,2,4-triazin (gelöst in 25 ml Aethanol) werden mit 2,44 g (0,02 Mol) Pyridin-3-carbaldehyd-N-oxid versetzt. Nach 1/2 stündigem Kochen unter Rückfluss wird das Reaktionsgemisch abgekühlt, der Festanteil abfiltriert, mit Aether gewaschen und getrocknet. Die so erhaltene Titelverbindung der Formel

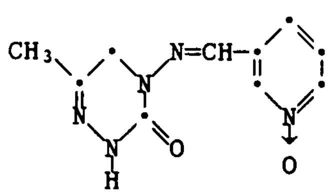

(Verbindung Nr. 4.09)
liegt als weisser Festkörper vor; Smp. 242-244 °C (Ausbeute: 3,6 g; 84 %).

Die vorstehend angegeben werden auch die folgenden Verbindungen der Formeln Ia, Ib und Ic hergestellt:

Tabelle 3

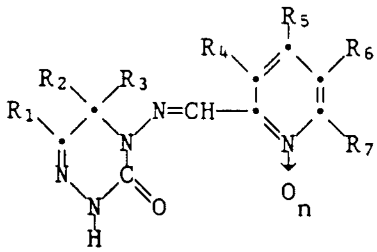

(Ia)

$R_4$, $R_5$, $R_6$, $R_7$ = H, n = 0

| Verb. Nr. | $R_1$ | $R_2$ | $R_3$ | physikalische Konstanten |
|---|---|---|---|---|
| 3.01. | $CH_3$ | H | H | Smp.: 249-250°C |
| 3.02. | $CH_3$ | $CH_3$ | $CH_3$ | Smp.: 162-163°C |

Tabelle 4

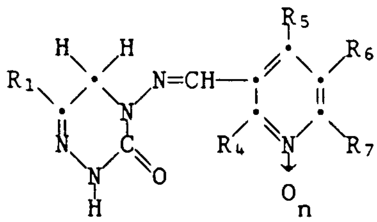

(Ib)

| Verb. Nr. | $R_1$ | $R_4$ | $R_5$ | $R_6$ | $R_7$ | n | physikalische Konstanten |
|---|---|---|---|---|---|---|---|
| 4.01 | $CH_3$ | H | H | H | Cl | 0 | Smp.: 227-228°C |
| 4.02 | $CH_3$ | Cl | H | Cl | Cl | 0 | Smp.: 248-249°C |
| 4.03 | $C_2H_5$ | H | H | H | Cl | 0 | Smp.: 226-227°C |
| 4.04 | $CH_3$ | H | H | H | $CH_3O$ | 0 | Smp.: 193-196°C |
| 4.05 | $CH_3$ | $CH_3O$ | H | H | $CH_3O$ | 0 | Smp.: 241-243°C |
| 4.06 | $CH_3$ | Cl | H | H | H | 0 | Smp.: 240-241°C |
| 4.07 | $CH_3$ | H | H | H | $CH_3$ | 0 | Smp.: 220-223°C |
| 4.08 | $CH_3$ | $NH_2$ | H | H | H | 0 | Smp.: > 250°C |
| 4.09 | $CH_3$ | H | H | H | H | 1 | Smp.: 242-244°C |
| 4.10 | $CH_3$ | H | H | $CH_3$ | H | 0 | Smp.: 229-230°C |
| 4.11 | $CH_3$ | H | $CH_3$ | H | H | 0 | |

Tabelle 4 (Fortsetzung)

| Verb. Nr. | $R_1$ | $R_4$ | $R_5$ | $R_6$ | $R_7$ | n | physikalische Konstanten |
|---|---|---|---|---|---|---|---|
| 4.12 | $CH_3$ | H | H | F | H | 0 | |
| 4.13 | $CH_3$ | F | H | H | H | 0 | |
| 4.14 | $CH_3$ | H | H | H | F | 0 | |
| 4.15 | $CH_3$ | H | F | H | H | 0 | |
| 4.16 | $CH_3$ | H | H | H | $CH_3S$ | 0 | |
| 4.17 | $CH_3$ | H | H | H | $(CH_3)_2N$ | 0 | |
| 4.18 | $CH_3$ | $CH_3$ | H | H | H | 1 | Smp.: 238-240°C |
| 4.19 | $CH_3$ | $CH_3$ | H | H | H | 0 | Smp.: 226-229°C |
| 4.20 | $CH_3$ | $SCH_3$ | H | H | H | 0 | Smp.: 255°C |
| 4.21 | $C_4H_9$(t) | H | H | H | H | 1 | Smp.: 129-132°C |
| 4.22 | $C_3H_7$(i) | H | H | H | H | 1 | Smp.: 120-121°C |

Tabelle 5

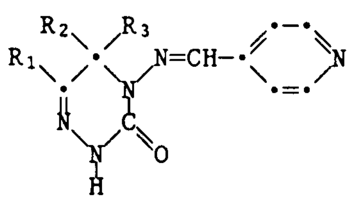

(Ic)

| Verb. Nr. | $R_1$ | $R_2$ | $R_3$ | physikalische Konstante |
|---|---|---|---|---|
| 5.01 | $CH_3$ | H | H | Smp.: 242°C |
| 5.02 | $(CH_3)_2CH$ | H | H | Smp.: 168-170°C |
| 5.03 | $CH_3$ | $CH_3$ | $CH_3$ | Smp.: 234-235°C |

2. Formulierungsbeispiele

Formulierungen für Wirkstoffe der Formel I resp. Kombinationen dieser Wirkstoffe mit andern Insektiziden oder Akariziden (% = Gewichtsprozent):

| F1. Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

| F2. Emulsions-Konzentrat | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff oder die Wirkstoffkombination mit dem Träger vermischt und auf einer geeigneten Mühle vermahlen wird.

| F4. Extruder-Granulat | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

| F5. Umhüllungs-Granulat | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

| F6. Suspensions-Konzentrat | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele

Beispiel B.1: Kontaktwirkung auf Aphis craccivora

In angezogene 4-5 Tage alte Erbsenkeimlinge (Pisum sativum) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 400 ppm der zu prüfenden Verbindung bis zur Tropfnässe direkt besprüht. Man verwendet pro Test-Verbindung zwei Pflanzen. Eine Auswertung der erzielten Abtötungsrate erfolgt nach weiteren 24 und 72 Stunden. Der Versuch wird bei 21-22 °C und einer rel. Luftfeuchtigkeit von etwa 55 % durchgeführt.

Verbindungen gemäss den Tabellen 3, 4 und 5 zeigen gute Wirkung in diesem Test.

Beispiel B.2: Systemische Wirkung auf Myzus Persicae

Peperonipflanzen werden mit gut besiedelten Erbsenkeimlingen infiziert. Die benutzte Erde wird 4 Tage später mit 25 ml 0,1%-iger Spritzbrühe, hergestellt aus einem 10%-igen Emulsionskonzentrat und Wasser angegossen, wodurch die Wirkstoffkonzentration 12,5 ppm (Gewichtsanteil bezogen auf das Erdvolumen) in der Erde beträgt. Um die Pflanzen wird eine Papiermanschette gelegt.

Die Auswertung der erzielten Abtötung erfolgt 3 und 7 Tagen nach Versuchsbeginn. Pro Testsubstanz werden zwei Pflanzen, je eine in einem separaten Topf, verwendet. Der Versuch wird bei 25 °C und ca. 65 % relativer Luftfeuchtigkeit durchgeführt.

Nach 1, 2 und 4 Wochen werden die Pflanzen neu besiedelt und jeweils nach 3 und 7 Tagen werden neue Auswertungen durchgeführt.

Verbindungen gemäss den Tabellen 3, 4 und 5 zeigen gute Wirkung in diesem Test.

Beispiel B.3: Kontaktwirkung auf Myzus persicae, Direktspraytest

Peperonipflanzen (im 6-Blattstadium, eingetopft) werden 4 Tage vor der Behandlung mit einer Population von Myzus Persicae (R-Stamm) infestiert, indem man 2-3 cm lange, mit Blattläusen gut besiedelte Erbsenkeimlinge auf die Peperonipflanzen legt. Sobald die Erbsenkeimlinge zu vertrocknen beginnen, wandern die Blattläuse auf die Versuchspflanzen (Peperoni) über. Die so behandelten Pflanzen werden 24 Stunden später mit einer, aus 25%-igem Spritzpulver hergestellten wässrigen Suspension enthaltend 100

ppm der zu prüfenden Verbindung bis zur Tropfnässe direkt besprüht. Man verwendet pro Testsubstanz vier Pflanzen. Eine Auswertung der erzielten Abtötungsrate erfolgt 7 Tage nach Applikation. Der Versuch wird bei 21-22 °C und etwa 60 % relativer Luftfeuchtigkeit durchgeführt.

Verbindungen gemäss den Tabellen 3, 4 und 5 zeigen in diesem Test eine gute Wirkung. Insbesondere Verbindung 4.09 zeigt eine Wirkung von über 80 % (Mortalität).

Beispiel B.4: Dauerwirkungstests Myzus persicae

Peperonipflanzen (im 6-Blattstadium, eingetopft) werden mit den Versuchslösungen mittels Sprayapplikation behandelt, 2 Tage nach der Behandlung werden die Versuchspflanzen mit einer Population von Myzus persicae (R-Stamm), wie in Beispiel B4 beschrieben, besiedelt. Die Bonitur erfolgt 5 Tage nach der Besiedlung auf % Mortalität.

Die Verbindungen gemäss den Tabellen 3, 4 und 5 zeigen eine gute Wirkung gegen Myzus persicae in diesem Test. Insbesondere Verbindung 4.09 zeigt eine Wirkung von über 80 % (Mortalität).

Beispiel B.5: Kontaktwirkung gegen Bemisia tabaci

Eingetopfte Bohnenpflanzen (Phaseolus vulgaris, Varietät Autan) werden im 2-Blattstadium mit Bemisia tabaci besiedelt (40 Adulten pro Pflanze). Nach 3tägiger Eiablage-Periode werden die Adulten entfernt. Zehn Tage nach Besiedelung als etwa 2/3 Nymphen im späten ersten Nymphenstadium, 1/3 schon im zweiten Nymphenstadium sind, werden die Pflanzen in einer Sprühkabine mit Spritzbrühe (hergestellt aus 10%-igem Emulsionskonzentrat und Wasser) in verschiedenen Konzentrationen behandelt.

Die Bonitur erfolgt etwa 24 Tage nach der Besiedlung auf tote Nymphen, Puppen und Adulte der $F_1$-Generation. Der Versuch wird bei 25 °C und 50-60 % relativer Luftfeuchtigkeit durchgeführt.
Verbindungen gemäss den Tabellen 3, 4 und 5 zeigen bei einer Konzentration von 3 ppm gute Wirkung. Insbesondere Verbindung 4.09 zeigt eine Wirkung von über 80 % (Mortalität).

**Patentansprüche**
**Patentansprüche für folgende Vertragsstaaten : AT, BE, CH, DE, DK, FR, GB, IT, LI, NL**

**1.** Verbindung der Formel

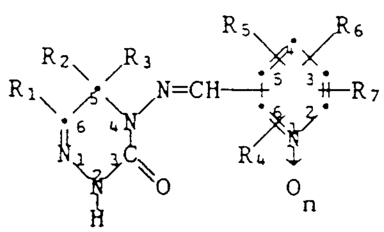

(I),

worin
$R_1$ $C_1$-$C_{12}$-Alkyl oder $C_3$-$C_7$-Cycloalkyl,
$R_2$ und $R_3$ voneinander unabhängig Wasserstoff oder $C_1$-$C_6$-Alkyl,
$R_4$, $R_5$, $R_6$ und $R_7$ voneinander unabhängig Wasserstoff, Halogen, $C_1$-$C_3$-Alkyl,

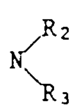

,

$C_1$-$C_3$-Alkoxy oder $C_1$-$C_3$-Alkylthio und n 0 oder 1
bedeuten, in freier Form oder in Salzform, mit der Massgabe, dass n für 1 steht, wenn gleichzeitig der Pyridinring über die 3-Position an die Methylidengruppe gebunden ist und $R_4$, $R_5$, $R_6$ und $R_7$ für Wasserstoff stehen.

**2.** Verbindung gemäss Anspruch 1 der Formel I, worin $R_1$ $C_1$-$C_4$-Alkyl bedeutet.

3. Verbindung gemäss Anspruch 1 der Formel I, worin $R_1$ Methyl, Ethyl, Isopropyl oder Cyclopropyl bedeutet.

4. Verbindung gemäss einem der Ansprüche 1 bis 3 der Formel I, worin n 1 bedeutet.

5. Verbindung gemäss einem der Ansprüche 1 bis 4 der Formel

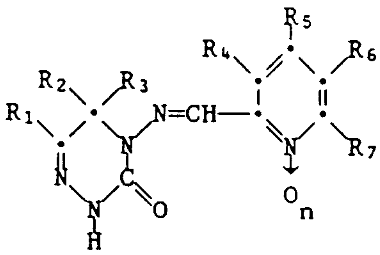

(Ia).

6. Verbindung gemäss einem der Ansprüche 1 bis 5 der Formel I oder Ia, worin $R_2$ und $R_3$ unabhängig voneinander Wasserstoff oder Methyl bedeuten.

7. Verbindung gemäss einem der Ansprüche 1 bis 6 der Formel I oder Ia, worin $R_2$ und $R_3$ Wasserstoff bedeuten.

8. Verbindung gemäss einem der Ansprüche 1 bis 4 der Formel

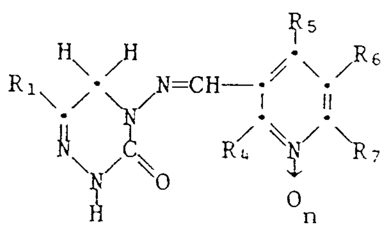

(Ib).

9. Verbindung gemäss einem der Ansprüche 1 bis 4, 6, 7 und 8 der Formel I, Ia oder Ib, worin $R_1$ Methyl oder Ethyl, $R_5$ Wasserstoff, $R_4$, $R_6$ und $R_7$ unabhängig voneinander Chlor, Methyl, Methoxy oder Amino bedeuten.

10. Verbindung gemäss einem der Ansprüche 1 bis 8 der Formel I, Ia oder Ib, worin $R_6$ Wasserstoff bedeutet.

11. Verbindung gemäss einem der Ansprüche 1 bis 4 und 6 bis 8 der Formel I, Ia oder Ib, worin $R_4$, $R_5$, $R_6$ und $R_7$ Wasserstoff bedeuten und n 1 bedeutet.

12. Verbindung gemäss einem der Ansprüche 1 bis 11 der Formel I, Ia oder Ib, worin $R_1$ Methyl bedeutet.

13. Verbindung gemäss Anspruch 8 der Formel Ib, worin $R_4$, $R_5$ und $R_6$ voneinander unabhängig Methyl oder Fluor, $R_7$ Wasserstoff, Methylthio oder Dimethylamino und n 0 bedeuten.

**14.** Verbindung gemäss einem der Ansprüche 1 bis 3 der Formel

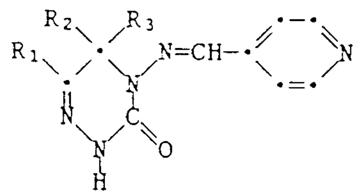

(Ic),

**15.** 2,3,4,5-Tetrahydro-3-oxo-4-[(pyridin-N-oxyd-3-yl)-methylenimino]-6-methyl-1,2,4-triazin der Formel

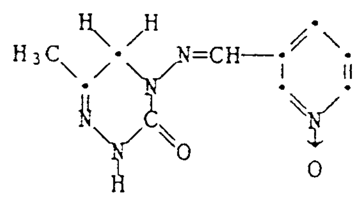

gemäss Anspruch 12.

**16.** Verfahren zur Herstellung einer Verbindung der Formel

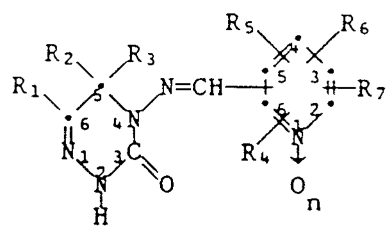

(I),

worin $R_1$, $R_2$, $R_3$, $R_4$, $R_5$, $R_6$, $R_7$ und n die in Anspruch 1 für die Formel I angegebenen Bedeutungen haben, in freier Form oder in Salzform,
dadurch gekennzeichnet, dass man
ein Aminotriazinon der Formel

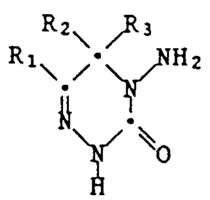

(II),

worin
$R_1$, $R_2$ und $R_3$ die in Anspruch 1 für die Formel I angegebenen Bedeutungen haben,
mit einem Aldehyd der Formel

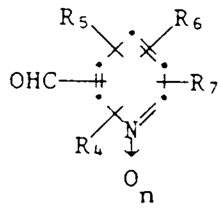

(III),

worin $R_4$, $R_5$, $R_6$, $R_7$ und n die in Anspruch 1 für die Formel I angegebenen Bedeutungen haben, wobei die gleiche Massgabe die in Anspruch 1 gilt, umsetzt und erwünschtenfalls die erhaltene Verbindung der Formel I in eines ihrer Salze umwandelt.

**17.** Schädlingsbekämpfungsmittel, welches eine Verbindung gemäss Anspruch 1 der Formel I als aktive Komponente sowie geeignete Träger und/oder Zuschlagsstoffe enthält.

**18.** Verwendung einer Verbindung, gemäss Anspruch 1 der Formel I zur Bekämpfung von Schädlingen.

**19.** Verwendung gemäss Anspruch 18 zur Bekämpfung von Insekten und Arachniden.

**20.** Verwendung gemäss Anspruch 19 zur Bekämpfung von pflanzenschädigenden Insekten.

**21.** Verwendung gemäss Anspruch 20 zur Bekämpfung von saugenden Insekten.

**22.** Verfahren zur Bekämpfung von Schädlingen, dadurch gekennzeichnet, dass man die Schädlinge bzw. deren verschiedene Entwicklungsstadien oder deren Aufenthaltsort mit einer pestizid wirksamen Menge einer Verbindung gemäss Anspruch 1 der Formel I oder mit einem Mittel, enthaltend neben Zusatz- und Trägerstoffen eine pestizid wirksame Menge einer solchen Verbindung, in Kontakt bringt oder behandelt.

**23.** Verfahren gemäss Anspruch 22 zur Bekämpfung von Insekten und Arachniden.

**24.** Verfahren gemäss Anspruch 23 zur Bekämpfung von pflanzenschädigenden Insekten.

**25.** Verfahren gemäss Anspruch 24 zur Bekämpfung von saugenden Insekten.

**Patentansprüche für folgenden Vertragsstaat : ES**

**1.** Verfahren zur Herstellung einer Verbindung der Formel

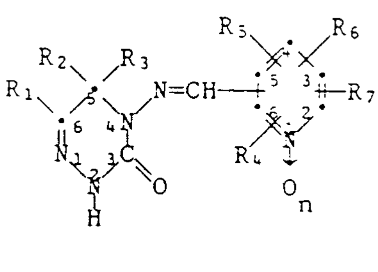

(I),

worin
$R_1$ $C_1$-$C_{12}$-Alkyl oder $C_3$-$C_7$-Cycloalkyl,
$R_2$ und $R_3$ voneinander unabhängig Wasserstoff oder $C_1$-$C_6$-Alkyl,
$R_4$, $R_5$, $R_6$ und $R_7$ voneinander unabhängig Wasserstoff, Halogen, $C_1$-$C_3$-Alkyl,

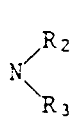

,

$C_1$-$C_3$-Alkoxy oder $C_1$-$C_3$-Alkylthio und n 0 oder 1
bedeuten, in freier Form oder in Salzform, mit der Massgabe, dass n für 1 steht, wenn gleichzeitig der Pyridinring über die 3-Position an die Methylidengruppe gebunden ist und $R_4$, $R_5$, $R_6$ und $R_7$ für Wasserstoff stehen, dadurch gekennzeichnet, dass man ein Aminotriazinon der Formel

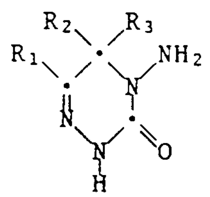

(II),

mit einem Aldehyd der Formel

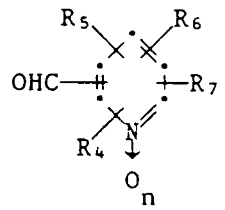

(III),

worin $R_1$ bis $R_7$ und n die für die Formel I angegebenen Bedeutungen haben, umsetzt und erwünschtenfalls die erhaltene Verbindung der Formel I in eines ihrer Salze umwandelt.

**2.** Verfahren gemäss Anspruch 1 zur Herstellung einer Verbindung der Formel I, worin $R_1$ $C_1$-$C_4$-Alkyl bedeutet.

**3.** Verfahren gemäss Anspruch 1 zur Herstellung einer Verbindung der Formel I, worin $R_1$ Methyl, Ethyl, Isopropyl oder Cyclopropyl bedeutet.

**4.** Verfahren gemäss einem der Ansprüche 1 bis 3 zur Herstellung einer Verbindung der Formel I, worin n 1 bedeutet.

**5.** Verfahren gemäss einem der Ansprüche 1 bis 4 zur Herstellung einer Verbindung der Formel

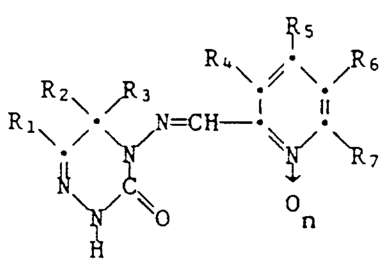

(Ia).

**6.** Verfahren gemäss einem der Ansprüche 1 bis 5 zur Herstellung einer Verbindung der Formel I oder Ia, worin $R_2$ und $R_3$ unabhängig voneinander Wasserstoff oder Methyl bedeuten.

**7.** Verfahren gemäss einem der Ansprüche 1 bis 6 zur Herstellung einer Verbindung der Formel I oder Ia, worin $R_2$ und $R_3$ Wasserstoff bedeuten.

8. Verfahren gemäss einem der Ansprüche 1 bis 4 zur Herstellung einer Verbindung der Formel

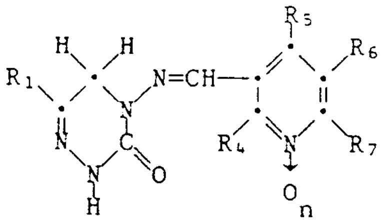

(Ib).

9. Verfahren gemäss einem der Ansprüche 1 bis 4, 6, 7 und 8 zur Herstellung einer Verbindung der Formel I, Ia oder Ib, worin $R_1$ Methyl oder Ethyl, $R_5$ Wasserstoff, $R_4$, $R_6$ und $R_7$ unabhängig voneinander Chlor, Methyl, Methoxy oder Amino bedeuten.

10. Verfahren gemäss einem der Ansprüche 1 bis 8 zur Herstellung einer Verbindung der Formel I, Ia oder Ib, worin $R_6$ Wasserstoff bedeutet.

11. Verfahren gemäss einem der Ansprüche 1 bis 4 und 6 bis 8 zur Herstellung einer Verbindung der Formel I, Ia oder Ib, worin $R_4$, $R_5$, $R_6$ und $R_7$ Wasserstoff bedeuten und n 1 bedeutet.

12. Verfahren gemäss einem der Ansprüche 1 bis 11 zur Herstellung einer Verbindung der Formel I, Ia oder Ib, worin $R_1$ Methyl bedeutet.

13. Verfahren gemäss Anspruch 8 zur Herstellung einer Verbindung der Formel Ib, worin $R_4$, $R_5$ und $R_6$ voneinander unabhängig Methyl oder Fluor, $R_7$ Wasserstoff, Methylthio oder Dimethylamino und n 0 bedeuten.

14. Verfahren gemäss einem der Ansprüche 1 bis 3 zur Herstellung einer Verbindung der Formel

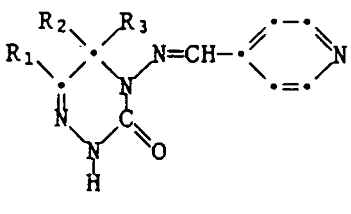

(Ic).

15. Verfahren gemäss Anspruch 12 zur Herstellung von 2,3,4,5-Tetrahydro-3-oxo-4-[(pyridin-N-oxyd-3-yl)-methylenimino]-6-methyl-1,2,4-triazin der Formel

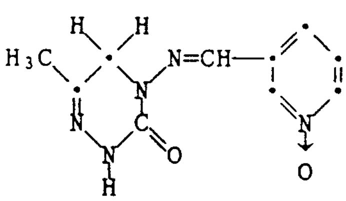

.

16. Schädlingsbekämpfungsmittel, welches eine gemäss Anspruch 1 erhältliche Verbindung der Formel I als aktive Komponente sowie geeignete Träger und/oder Zuschlagsstoffe enthält.

17. Verwendung einer gemäss Anspruch 1 erhältlichen Verbindung der Formel I zur Bekämpfung von Schädlingen.

**18.** Verwendung gemäss Anspruch 17 zur Bekämpfung von Insekten und Arachniden.

**19.** Verwendung gemäss Anspruch 18 zur Bekämpfung von pflanzenschädigenden Insekten.

**20.** Verwendung gemäss Anspruch 19 zur Bekämpfung von saugenden Insekten.

**21.** Verfahren zur Bekämpfung von Schädlingen, dadurch gekennzeichnet, dass man die Schädlinge bzw. deren verschiedene Entwicklungsstadien oder deren Aufenthaltsort mit einer pestizid wirksamen Menge einer gemäss Anspruch 1 erhältlichen Verbindung der Formel I oder mit einem Mittel, enthaltend neben Zusatz- und Trägerstoffen eine pestizid wirksame Menge einer solchen Verbindung, in Kontakt bringt oder behandelt.

**22.** Verfahren gemäss Anspruch 21 zur Bekämpfung von Insekten und Arachniden.

**23.** Verfahren gemäss Anspruch 22 zur Bekämpfung von pflanzenschädigenden Insekten.

**24.** Verfahren gemäss Anspruch 23 zur Bekämpfung von saugenden Insekten.

**Claims**
**Claims for the following Contracting States : AT, BE, CH, DE, DK, FR, GB, IT, LI, NL**

**1.** A compound of formula

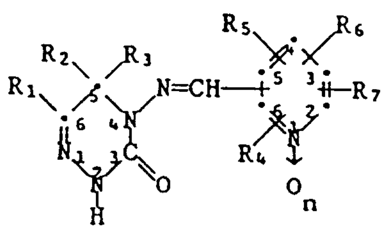

(I)

wherein
$R_1$ is $C_1$-$C_{12}$alkyl or $C_3$-$C_7$cycloalkyl,
$R_2$ and $R_3$ are each independently of the other hydrogen or $C_1$-$C_6$alkyl,
$R_4$, $R_5$, $R_6$ and $R_7$ are each independently of the others hydrogen, halogen, $C_1$-$C_3$alkyl,

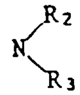,

$C_1$-$C_3$alkoxy or $C_1$-$C_3$alkylthio, and
n is 0 or 1,
in free form or in salt form, with the proviso that n is 1 if simultaneously the pyridine ring is attached via the 3-position to the methylidene group and $R_4$, $R_5$, $R_6$ and $R_7$ are each hydrogen.

**2.** A compound according to claim 1 of formula I, wherein $R_1$ is $C_1$-$C_4$alkyl.

**3.** A compound according to claim 1 of formula I, wherein $R_1$ is methyl, ethyl, isopropyl or cyclopropyl.

**4.** A compound according to any one of claims 1 to 3 of formula I, wherein n is 1.

**5.** A compound according to any one of claims 1 to 4 of formula

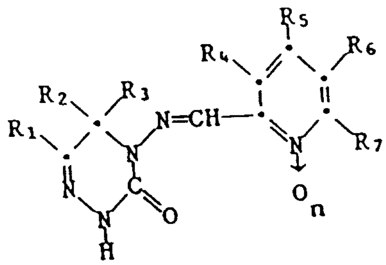

(Ia).

**6.** A compound according to any one of claims 1 to 5 of formula I or Ia, wherein $R_2$ and $R_3$ are each independently of the other hydrogen or methyl.

**7.** A compound according to any one of claims 1 to 6 of formula I or Ia, wherein $R_2$ and $R_3$ are each hydrogen.

**8.** A compound according to any one of claims 1 to 4 of formula

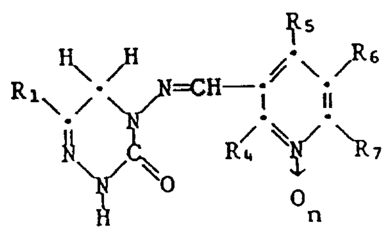

(Ib).

**9.** A compound according to any one of claims 1 to 4, 6, 7 and 8 of formula I, Ia or Ib, wherein $R_1$ is methyl or ethyl, $R_5$ is hydrogen, and $R_4$, $R_6$ and $R_7$ are each independently of the others chlorine, methyl, methoxy or amino.

**10.** A compound according to any one of claims 1 to 8 of formula I, Ia or Ib, wherein $R_6$ is hydrogen.

**11.** A compound according to any one of claims 1 to 4 and 6 to 8 of formula I, Ia or Ib, wherein $R_4$, $R_5$, $R_6$ and $R_7$ are each hydrogen and n is 1.

**12.** A compound according to any one of claims 1 to 11 of formula I, Ia or Ib, wherein $R_1$ is methyl.

**13.** A compound according to claim 8 of formula Ib, wherein $R_4$, $R_5$ and $R_6$ are each independently of the others methyl or fluorine, $R_7$ is hydrogen, methylthio or dimethylamino, and n is 0.

**14.** A compound according to any one of claims 1 to 3 of formula

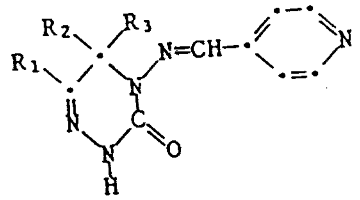

(Ic).

**15.** 2,3,4,5-Tetrahydro-3-oxo-4-[(pyridine-N-oxid-3-yl)methyleneimino]-6-methyl-1,2,4-triazine of formula

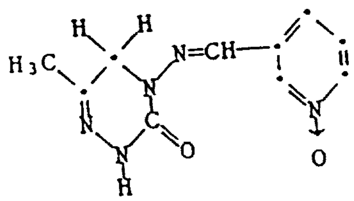

according to claim 12.

**16.** A process for the preparation of a compound of formula

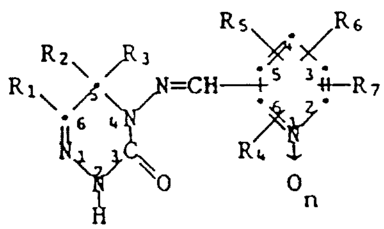

(I),

wherein $R_1$, $R_2$, $R_3$, $R_4$, $R_5$, $R_6$, $R_7$ and n have the meanings given in claim 1 for formula I, in free form or in salt form, which comprises reacting an aminotriazinone of formula

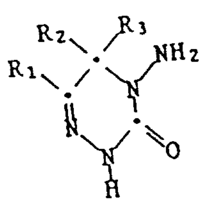

(II),

wherein
$R_1$, $R_2$ and $R_3$ have the meanings given in claim 1 for formula I,
with an aldehyde of formula

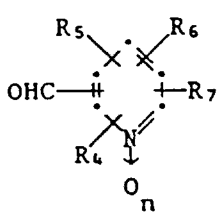

(III),

wherein
$R_4$, $R_5$, $R_6$, $R_7$ and n have the meanings given in claim 1 for formula I, with the same proviso as in claim 1, and, if desired, converting the resultant compound of formula I into one of its salts.

**17.** A pesticidal composition that comprises a compound according to claim 1 of formula I as active component, and also suitable carriers and/or adjuvants.

**18.** Use of a compound according to claim 1 of formula I for controlling pests.

**19.** Use according to claim 18 for controlling insects and arachnids.

20. Use according to claim 19 for controlling plant-injurious insects.

21. Use according to claim 20 for controlling sucking insects.

22. A method of controlling pests, which comprises treating or bringing the pests, their different development stages or the locus thereof, into contact with a pesticidally effective amount of a compound according to claim 1 of formula I, or with a composition comprising a pesticidally effective amount of such a compound in addition to adjuvants and carriers.

23. A method according to claim 22 for controlling insects and arachnids.

24. A method according to claim 23 for controlling plant-injurious insects.

25. A method according to claim 24 for controlling sucking insects.

**Claims for the following Contracting State : ES**

1. A process for the preparation of a compound of formula

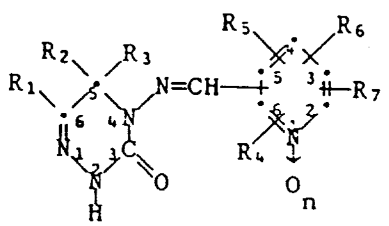

(I)

wherein
$R_1$ is $C_1$-$C_{12}$alkyl or $C_3$-$C_7$cycloalkyl,
$R_2$ and $R_3$ are each independently of the other hydrogen or $C_1$-$C_6$alkyl,
$R_4$, $R_5$, $R_6$ and $R_7$ are each independently of the others hydrogen, halogen, $C_1$-$C_3$alkyl,

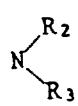

,

$C_1$-$C_3$alkoxy or $C_1$-$C_3$alkylthio, and
n is 0 or 1,
in free form or in salt form, with the proviso that n is 1 if simultaneously the pyridine ring is attached via the 3-position to the methylidene group and $R_4$, $R_5$, $R_6$ and $R_7$ are each hydrogen, which process comprises reacting an aminotriazinone of formula

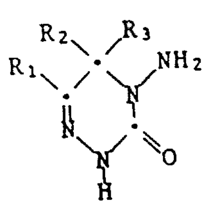

(II),

with an aldehyde of formula

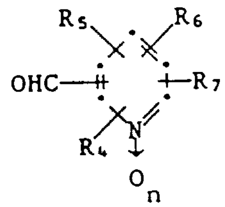

(III),

in which formulae $R_1$ to $R_7$ and n have the meanings given for formula I, and, if desired, converting the resultant compound of formula I into one of its salts.

2. A process according to claim 1 for the preparation of a compound of formula I, wherein $R_1$ is $C_1$-$C_4$alkyl.

3. A process according to claim 1 for the preparation of a compound of formula I, wherein $R_1$ is methyl, ethyl, isopropyl or cyclopropyl.

4. A process according to any one of claims 1 to 3 for the preparation of a compound of formula I, wherein n is 1.

5. A process according to any one of claims 1 to 4 for the preparation of a compound of formula

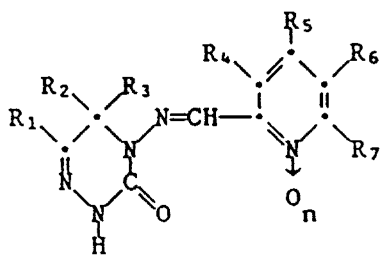

(Ia).

6. A process according to any one of claims 1 to 5 for the preparation of a compound of formula I or Ia, wherein $R_2$ and $R_3$ are each independently of the other hydrogen or methyl.

7. A process according to any one of claims 1 to 6 for the preparation of a compound of formula I or Ia, wherein $R_2$ and $R_3$ are each hydrogen.

8. A process according to any one of claims 1 to 4 for the preparation of a compound of formula

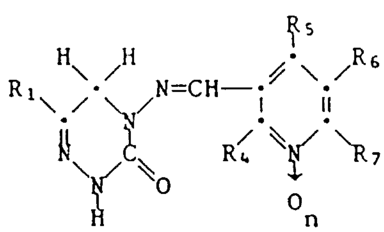

(Ib).

9. A process according to any one of claims 1 to 4, 6, 7 and 8 for the preparation of a compound of formula I, Ia or Ib, wherein $R_1$ is methyl or ethyl, $R_5$ is hydrogen, and $R_4$, $R_6$ and $R_7$ are each independently of the others chlorine, methyl, methoxy or amino.

10. A process according to any one of claims 1 to 8 for the preparation of a compound formula I, Ia or Ib, wherein $R_6$ is hydrogen.

**11.** A process according to any one of claims 1 to 4 and 6 to 8 for the preparation of a compound of formula I, Ia or Ib, wherein $R_4$, $R_5$, $R_6$ and $R_7$ are each hydrogen and n is 1.

**12.** A process according to any one of claims 1 to 11 for the preparation of a compound of formula I, Ia or Ib, wherein $R_1$ is methyl.

**13.** A process according to claim 8 for the preparation of a compound of formula Ib, wherein $R_4$, $R_5$ and $R_6$ are each independently of the others methyl or fluorine, $R_7$ is hydrogen, methylthio or dimethylamino, and n is 0.

**14.** A process according to any one of claims 1 to 3 for the preparation of a compound of formula

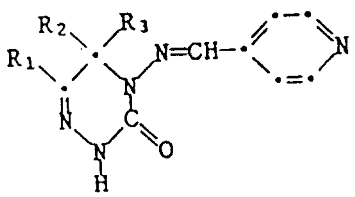

(Ic).

**15.** A process according to claim 12 for the preparation of 2,3,4,5-tetrahydro-3-oxo-4-[(pyridine-N-oxid-3-yl)-methyleneimino]-6-methyl-1,2,4-triazine of formula

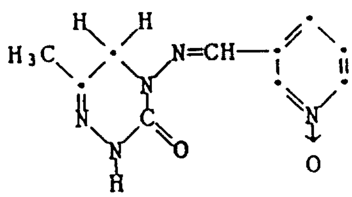

.

**16.** A pesticidal composition that comprises a compound of formula I obtainable in accordance with claim 1 as active component, and also suitable carriers and/or adjuvants.

**17.** Use of a compound of formula I obtainable in accordance with claim 1 for controlling pests.

**18.** Use according to claim 17 for controlling insects and arachnids.

**19.** Use according to claim 18 for controlling plant-injurious insects.

**20.** Use according to claim 19 for controlling sucking insects.

**21.** A method of controlling pests, which comprises treating or bringing the pests, their different development stages or the locus thereof, into contact with a pesticidally effective amount of a compound of formula I obtainable in accordance with claim 1, or with a composition comprising a pesticidally effective amount of such a compound in addition to adjuvants and carriers.

**22.** A method according to claim 21 for controlling insects and arachnids.

**23.** A method according to claim 22 for controlling plant-injurious insects.

**24.** A method according to claim 23 for controlling sucking insects.

**Revendications**

**Revendications pour les Etats contractants suivants : AT, BE, CH, DE, DK, FR, GB, IT, LI, NL**

**1.** Composé de formule

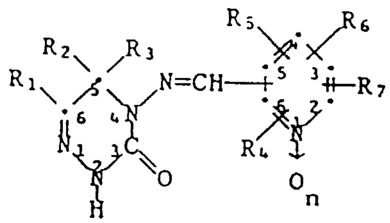

(I),

dans laquelle
$R_1$ représente un groupe alkyle en C1-C12 ou cycloalkyle en C3-C7,
$R_2$ et $R_3$ représentent chacun, indépendamment l'un de l'autre, l'hydrogène ou un groupe alkyle en C1-C6,
$R_4$, $R_5$, $R_6$ et $R_7$ représentent chacun, indépendamment les uns des autres, l'hydrogène, un halogène, un groupe alkyle en C1-C3,

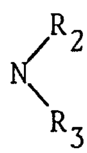

,

alcoxy en C1-C3 ou alkylthio en C1-C3 et n est égal à 0 ou 1,
à l'état libre ou à l'état de sel, sous réserve que n est égal à 1 lorsque, simultanément, le cycle pyridine est relié au groupe méthylidène en position 3 et $R_4$, $R_5$, $R_6$ et $R_7$ représentent l'hydrogène.

**2.** Composé de formule I selon revendication 1, dans laquelle $R_1$ représente un groupe alkyle en C1-C4.

**3.** Composé de formule I selon revendication 1, dans laquelle $R_1$ représente un groupe méthyle, éthyle, isopropyle ou cyclopropyle.

**4.** Composé de formule I selon une des revendications 1 à 3, dans laquelle n est égal à 1.

**5.** Composé de formule I selon une des revendications 1 à 4, de formule

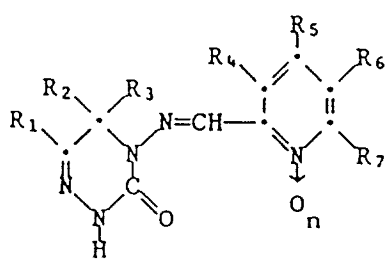

(Ia)

**6.** Composé selon une des revendications 1 à 5, de formule I ou Ia dans laquelle $R_2$ et $R_3$ représentent chacun, indépendamment l'un de l'autre, l'hydrogène ou un groupe méthyle.

**7.** Composé selon une des revendications 1 à 6, de formule I ou Ia, dans laquelle $R_2$ et $R_3$ représentent l'hydrogène.

**8.** Composé selon une des revendications 1 à 4, de formule

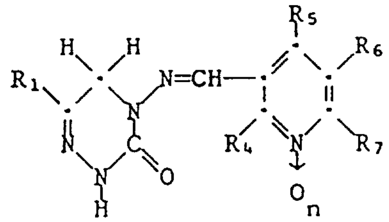

(Ib)

**9.** Composé selon une des revendications 1 à 4, 6, 7 et 8, de formule I, Ia ou Ib, dans laquelle $R_1$ représente un groupe méthyle ou éthyle, $R_5$ l'hydrogène, $R_4$, $R_6$ et $R_7$, indépendamment les uns des autres, le chlore, un groupe méthyle, méthoxy ou amino.

**10.** Composé selon une des revendications 1 à 8, de formule I, Ia ou Ib, dans laquelle $R_6$ représente l'hydrogène.

**11.** Composé selon une des revendications 1 à 4 et 6 à 8, de formule I, Ia ou Ib, dans laquelle $R_4$, $R_5$, $R_6$ et $R_7$ représentent l'hydrogène et n est égal à 1.

**12.** Composé selon une des revendications 1 à 11, de formule I, Ia ou Ib, dans laquelle $R_1$ représente un groupe méthyle.

**13.** Composé selon revendication 8, de formule Ib dans laquelle $R_4$, $R_5$ et $R_6$ représentent chacun, indépendamment les uns des autres, un groupe méthyle ou le fluor, $R_7$ représente l'hydrogène, un groupe méthylthio ou diméthylamino et n est égal à 0.

**14.** Composé selon une des revendications 1 à 3, de formule

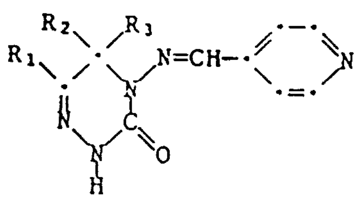

(Ic),

**15.** La 2,3,4,5-tétrahydro-3-oxo-4[(pyridine-N-oxydo-3-yl)-méthylène-imino]-6-méthyl-1,2,4-triazine de formule

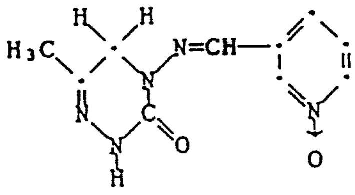

selon revendication 12.

**16.** Procédé de préparation d'un composé de formule

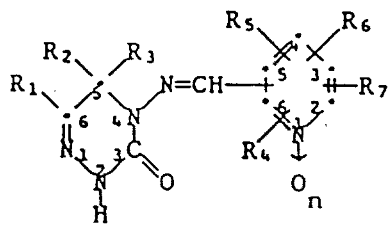

(I),

dans laquelle $R_1$, $R_2$, $R_3$, $R_4$, $R_5$, $R_6$, $R_7$ et n ont les significations indiquées en référence à la formule I dans la revendication 1,
caractérisé en ce que
on fait réagir une aminotriazinone de formule

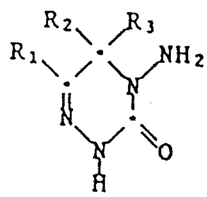

(II),

dans laquelle
$R_1$, $R_2$ et $R_3$ ont les significations indiquées en référence à la formule I dans la revendication 1,
avec un aldéhyde de formule

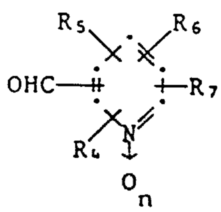

(III),

dans laquelle
$R_4$, $R_5$, $R_6$, $R_7$ et n ont les significations indiquées en référence à la formule I dans la revendication 1, avec la réserve indiquée dans la revendication 1,
et si on le désire, on convertit le composé ainsi obtenu, répondant à la formule I, en l'un de ses sels.

**17.** Produit parasiticide contenant en tant que composant actif un composé de formule I selon revendication 1 et des véhicules et/ou additifs appropriés.

**18.** Utilisation d'un composé de formule I selon revendication 1 pour la lutte contre les parasites.

**19.** Utilisation selon revendication 18, pour la lutte contre les insectes et les arachnides.

**20.** Utilisation selon revendication 19, pour la lutte contre les insectes nuisibles pour les végétaux.

**21.** Utilisation selon revendication 20, pour la lutte contre les insectes suçeurs.

**22.** Procédé pour combattre les parasites, caractérisé en ce que l'on traite ou l'on met en contact les parasites ou leurs divers stades de développement ou leur habitat avec une quantité parasiticide efficace d'un composé de formule I selon revendication 1, ou d'un produit contenant, avec des véhicules et additifs, une quantité parasiticide efficace d'un tel composé.

**23.** Procédé selon revendication 22, pour la lutte contre les insectes et les arachnides.

**24.** Procédé selon revendication 23, pour la lutte contre les insectes nuisibles pour les végétaux.

**25.** Procédé selon revendication 24, pour la lutte contre les insectes suçeurs.

**Revendications pour l'Etat contractant suivant : ES**

**1.** Procédé de préparation d'un composé de formule

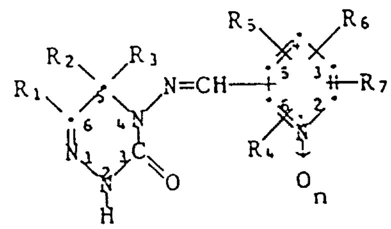

(I),

dans laquelle
$R_1$ représente un groupe alkyle en C1-C12 ou cycloalkyle en C3-C7,
$R_2$ et $R_3$ représentent chacun, indépendamment l'un de l'autre, l'hydrogène ou un groupe alkyle en C1-C6,
$R_4$, $R_5$, $R_6$ et $R_7$ représentent chacun, indépendamment les uns des autres, l'hydrogène, un halogène, un groupe alkyle en C1-C3,

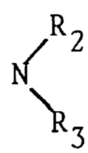

,

alcoxy en C1-C3 ou alkylthio en C1-C3 et n est égal à 0 ou 1,
à l'état libre ou à l'état de sel, sous réserve que n est égal à 1 lorsque, simultanément, le cycle pyridine est relié en position 3 au groupe méthylidène et $R_4$, $R_5$, $R_6$ et $R_7$ représentent l'hydrogène, caractérisé en ce que l'on fait réagir une aminotriazinone de formule

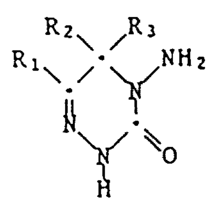

(II),

avec un aldéhyde de formule

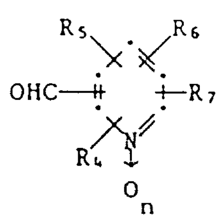

(III),

dans lesquelles $R_1$ à $R_7$ et n ont les significations indiquées en référence à la formule I, et si on le

désire, on convertit un composé ainsi obtenu, répondant à la formule I, en l'un de ses sels.

2. Procédé selon revendication 1, pour la préparation d'un composé de formule I dans laquelle $R_1$ représente un groupe alkyle en C1-C4.

3. Procédé selon revendication 1, pour la préparation d'un composé de formule I dans laquelle $R_1$ représente un groupe méthyle, éthyle, isopropyle ou cyclopropyle.

4. Procédé selon l'une des revendications 1 à 3, pour la préparation d'un composé de formule I dans laquelle n est égal à 1.

5. Procédé selon l'une des revendications 1 à 4, pour la préparation d'un composé de formule

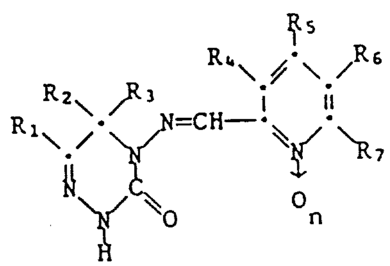

(Ia)

6. Procédé selon l'une des revendications 1 à 5, pour la préparation d'un composé de formule I ou Ia dans laquelle $R_2$ et $R_3$ représentent chacun, indépendamment l'un de l'autre, l'hydrogène ou un groupe méthyle.

7. Procédé selon l'une des revendications 1 à 6, pour la préparation d'un composé de formule I ou Ia dans laquelle $R_2$ et $R_3$ représentent l'hydrogène.

8. Procédé selon une des revendications 1 à 4, pour la préparation d'un composé de formule

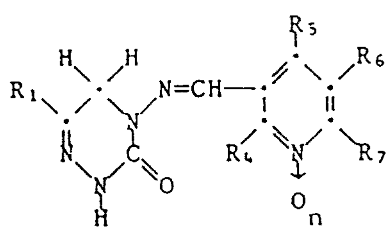

(Ib)

9. Procédé selon l'une des revendications 1 à 4, 6, 7 et 8, pour la préparation d'un composé de formule I, Ia ou Ib dans laquelle $R_1$ représente un groupe méthyle ou éthyle, $R_5$ l'hydrogène et $R_4$, $R_6$ et $R_7$, indépendamment les uns des autres, le chlore, un groupe méthyle, méthoxy ou amino.

10. Procédé selon l'une des revendications 1 à 8, pour la préparation d'un composé de formule I, Ia ou Ib dans laquelle $R_6$ représente l'hydrogène.

11. Procédé selon l'une des revendications 1 à 4 et 6 à 8, pour la préparation d'un composé de formule I, Ia ou Ib dans laquelle $R_4$, $R_5$, $R_6$ et $R_7$ représentent l'hydrogène et n est égal à 1.

12. Procédé selon l'une des revendications 1 à 11, pour la préparation d'un composé de formule I, Ia ou Ib dans laquelle $R_1$ représente un groupe méthyle.

**13.** Procédé selon revendication 8, pour la préparation d'un composé de formule Ib, dans laquelle $R_4$, $R_5$ et $R_6$ représentent chacun, indépendamment les uns des autres, un groupe méthyle ou le fluor, $R_7$ l'hydrogène, un groupe méthylthio ou diméthylamino, et n = 0.

**14.** Procédé selon l'une des revendications 1 à 3, pour la préparation d'un composé de formule

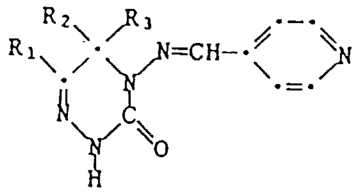

(Ic).

**15.** Procédé selon revendication 12, pour la préparation de la 2,3,4,5-tétrahydro-3-oxo-4-[(pyridine-N-oxydo-3-yl)-méthylène-imino]-6-méthyl-1,2,4-triazine de formule

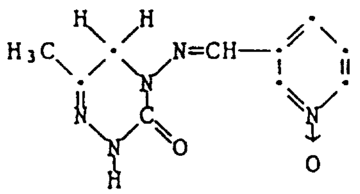

**16.** Produit parasiticide contenant en tant que composant actif un composé de formule I obtenu selon revendication 1, avec des véhicules et/ou additifs appropriés.

**17.** Utilisation d'un composé de formule I obtenu selon revendication 1, pour la lutte contre les parasites.

**18.** Utilisation selon revendication 17, pour la lutte contre les insectes et les arachnides.

**19.** Utilisation selon revendication 18, pour la lutte contre les insectes nuisibles pour les végétaux.

**20.** Utilisation selon revendication 19, pour la lutte contre les insectes suçeurs.

**21.** Procédé pour combattre les parasites, caractérisé en ce que l'on met en contact ou l'on traite les parasites ou leurs divers stades de développement ou leur habitat avec une quantité parasiticide efficace d'un composé de formule I obtenu selon revendication 1 ou d'un produit contenant, avec des véhicules et additifs, une quantité parasiticide efficace d'un tel composé.

**22.** Procédé selon revendication 21, pour la lutte contre les insectes et les arachnides.

**23.** Procédé selon revendication 22, pour la lutte contre les insectes nuisibles pour les végétaux.

**24.** Procédé selon revendication 23, pour la lutte contre les insectes suçeurs.